# EUROPEAN PATENT SPECIFICATION

(11) **EP 2 759 341 B1**
(45) Date of publication and mention of the grant of the patent: **11.03.2020**
(21) Application number: 12834431.4
(22) Date of filing: 24.09.2012
(51) Int. Cl.: B01J 31/16, B01J 37/20, C07C 209/26, C07C 211/51, B01J 23/44, B01J 27/045, B01J 31/22

(54) **METHOD FOR PREPARING P-PHENYLENEDIAMINE ANTIOXIDANT USING A SULFUR-CONTAINING PALLADIUM/CARBON CATALYST**
VERFAHREN ZUR HERSTELLUNG EINES P-PHENYLENDIAMIN-ANTIOXIDANS UNTER VERWENDUNG EINES SCHWEFELHALTIGER PALLADIUM-/KOHLENSTOFFKATALYSATORS
PROCÉDÉ POUR LA PRÉPARATION D'ANTIOXYDANT P-PHÉNYLÉNEDIAMINE EN UTILISANT UN CATALYSEUR PALLADIUM SUR CHARBON CONTENANT DU SOUFRE

(30) Priority: 22.09.2011 CN 201110284001
(43) Date of publication of application: 30.07.2014
(73) Proprietor: Sennics Co., Ltd., China (Shanghai) Pilot Free Trade Zone 200120 (CN)
(72) Inventor: LI, Xiaonian, Shanghai 201204 (CN); ZHANG, Qunfeng, Shanghai 201204 (CN); CHEN, Xinmin, Shanghai 201204 (CN); FENG, Feng, Shanghai 201204 (CN); MA, Lei, Shanghai 201204 (CN); LU, Chunshan, Shanghai 201204 (CN); LI, Chunsheng, Shanghai 201204 (CN)
(74) Representative: Novagraaf Technologies
(86) International application number: PCT/CN2012/081859
(87) International publication number: WO 2013/041061

(56) References cited:
- CN-A- 1 170 711
- CN-A- 1 475 475
- CN-A- 101 733 169
- CN-A- 101 733 169
- CN-A- 102 146 042
- US-A- 4 043 942
- US-A- 4 210 602
- US-A- 4 210 602
- US-A1- 2007 155 617

## Description

### Technical field of the invention

The present invention relates to the field of p-phenylenediamine (PPD) antioxidants, and in particular to a method for preparing PPD antioxidant.

### Background of the invention

PPD antioxidants are widely used because of their favourable protecting performance, and an important industrial synthesis method of PPD antioxidants is reductive alkylation which is a synthesis method using 4-aminodiphenylamine (4-ADPA) (RT base) and an aliphatic ketone as raw materials. For example, rubber antioxidant 4020, also known as antioxidant DMBPPD, chemical name N-(1,3-dimethylbutyl)-N'-phenyl-para-phenylenediamine (6PPD), plays an important role in aspects including ozone resistance, oxidization resistance, thermal resistance, and flex cracking resistance etc. and has been widely used as the popularity of meridian tires arises. Rubber antioxidant 4020 may be prepared by hydrogenation dehydration of raw materials N-phenyl-p-phenylene diamine (RT base) and methylisobutyl ketone (MIBK) in the presence of a catalyst according to the following reaction equation:

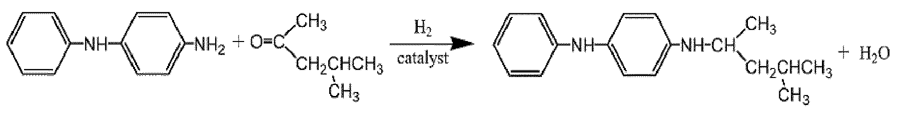

The hydrogenation reductive alkylation may also be a two-step process consisting of Step 1: RT base and MIBK react through dehydration condensation to form an imine, and Step 2: the generated imine is reduced by hydrogen to 4020, according to the following reaction equations:

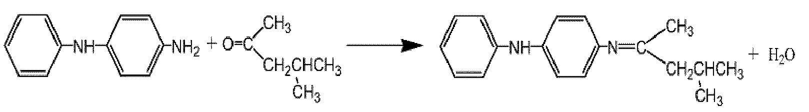

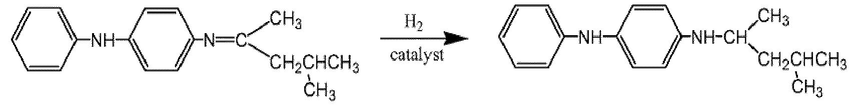

In the reaction, step 1 may be conducted in the presence of a proton acid catalyst or be conducted spontaneously without a catalyst and Step 2 can be carried out in the presence of a hydrogenation catalyst with relatively high selectivity.

In the hydrogenation reductive alkylation for preparing the PPD rubber antioxidant, major side reactions include hydrogenation of a raw material to form a corresponding alcohol, hydrogenolysis of raw materials or products, hydrogenation of a benzene ring, and tar generated by overheating etc. The key to synthesize a PPD rubber antioxidant with good quality and low cost is to use a catalyst with good activity and selectivity, and can be recycled and reused.

Currently, catalysts for making PPD rubber antioxidants in the industry include copper-based catalysts and platinum-carbon catalysts. Patent No. 200610161327.2 discloses a method for preparing a Cu-Zn/Al₂O₃ catalyst for preparing 4020 through reductive alkylation. The copper-based catalyst is relatively cheap, but not satisfactory in selectively. Because a large amount of MIBK is hydrogenated and reduced to a corresponding alcohol methylisobutyl alcohol (MIBA), and the percentage of MIBA at the end of the reaction can reach between 9.3% to 97.4% of the total amount of MIBK and MIBA. As a result, the raw materials are wasted, and the cost is increased. In addition, the metal platinum in a conventional platinum-carbon catalyst is very expensive, and product cost will be greatly increased by mass industrial use of the catalyst. CN-A-101 733 169 discloses a method for preparing p-phenylenediamine compounds by the reaction of 4-aminodiphenylamine and a ketone using a copper catalyst passivated with a sulphur compound or a halogen.

Pd has been commonly used as a hydrogenation catalyst and widely used in the hydrogenation reduction of radicals including a nitro group, a carbonyl group, a carbon-carbon double bond, and a carbon-nitrogen double bond etc. However, when palladium (Pd)/active carbon catalyst is applied to preparing 4020 through reductive alkylation, raw materials and products will have serious side reactions of hydrogenolysis of C-N bonds so that the selectivity for the target product is very low.

To this date, Pd has not been successfully used as an active component in the catalyst for preparing a PPD rubber antioxidant with a high conversion rate and high selectivity, basically because it is difficult to control side reactions of C-N bond hydrogenolysis caused by the application of the Pd catalyst.

### Summary of the invention

In order to solve the problems that an existing copper-based catalyst is not satisfactory in selectivity while a platinum-carbon catalyst is expensive, the present invention uses a sulfur-containing Pd/carbon catalyst. A method for preparing a sulfur-containing Pd/carbon catalyst includes the following steps:
loading Pd on an active carbon to obtain a Pd/carbon catalyst;
mixing the Pd/carbon catalyst with a solvent to obtain a slurry;
adding a sulfide into the slurry, and stirring thoroughly at a predetermined temperature;
filtering to obtain the sulfur-containing Pd/carbon catalyst.

Preferably, in the step of adding the sulfide to the slurry, the molar ratio of the added sulfide to Pd loaded on the Pd/carbon catalyst is 0.1 to 10:1. More preferably, the molar ratio of the added sulfide to Pd loaded on the Pd/carbon catalyst is 0.1 to 1:1.

Preferably, the sulfide adopted in the preparation process may be selected from one or more in a group consisting of thiol, thioether, alkyl disulfide, thiofuran, hydrogen sulfide, ammonium sulfide, ammonium hydrosulfide, a sulfide of an alkaline metal, and a hydrosulfide of an alkaline metal.

More preferably, the sulfide is selected from one or more in a group consisting of methyl mercaptan, ethyl thiol, methyl thioether, ethyl thioether, diphenyl thioether, dimethyl disulfide, thiofuran, hydrogen sulfide, ammonium sulfide, ammonium hydrosulfide, sodium sulfide, and potassium hydrosulfide. In a specific embodiment provided by the present invention, the adopted sulfide is diphenyl thioether or dimethyl disulfide.

In the preparation method the adopted solvent may be methanol, ethanol, acetone, MIBK or water.

In the preparation method preferably, the granularity of the adopted active carbon is 50 to 1000 meshes; the special surface area is 600 to 1800 m²/g, and the loading amount of Pd is 0.5 to 10wt%.

The present invention provides a method for preparing a PPD rubber antioxidant. The method uses 4-ADPA and an aliphatic ketone as raw materials, and the sulfur-containing Pd/carbon catalyst as a catalyst to prepare a PPD compound. Preferably, the sulfur-containing Pd/carbon catalyst is added to the reaction system in an amount such that the weight ratio of Pd loaded on the sulfur-containing Pd/carbon catalyst to 4-ADPA is 0.01 to 1 wt %.

Preferably, the method for preparing the PPD rubber antioxidant includes the following steps: use 4-ADPA and the aliphatic ketone as the raw materials, add the sulfur-containing Pd/carbon catalyst directly into the reaction system while stirring, introduce a predetermined amount of hydrogen, and synthesize the PPD rubber antioxidant through liquid phase hydrogenation.

Different from the steps above, the method for preparing the PPD rubber antioxidant of the present invention may be implemented by the following steps: use 4-ADPA and the aliphatic ketone as the raw materials and a proton acid or active carbon as a catalyst, perform dehydration condensation at 120°C to 150°C while stirring to synthesize an intermediate product; add the sulfur-containing Pd/carbon catalyst into a reaction system formed by the intermediate product and a solvent, introduce a predetermined amount of hydrogen, and synthesize the PPD rubber antioxidant through liquid phase hydrogenation.

The sulfur-containing Pd/carbon catalyst prepared by the preparation method provided above exhibits good activity, selectivity, and indiscriminately using performance. Applied in preparing the PPD rubber antioxidant, the sulfur-containing Pd/carbon catalyst not only improves the yield of the PPD rubber antioxidant and inhibits the raw materials from being reduced into corresponding alcohols, but also improves the selectivity of the reaction.

### Brief description of the drawings

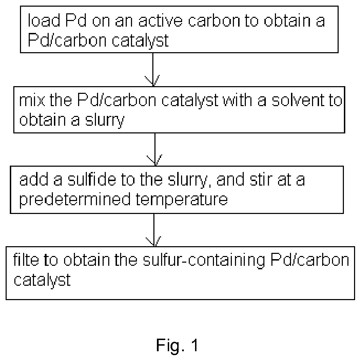

Fig. 1 shows a flowchart of method for preparing a sulfur-containing Pd/carbon catalyst provided by the present invention.

### Detailed description of the invention

Embodiments of the present invention will be described in details hereinafter. However, the following embodiments are only used for understanding the present invention, instead of limiting the present invention. The present invention may be implemented by various different methods limited and covered by the claims.

Fig. 1 shows a method for preparing a sulfur-containing Pd/carbon catalyst including the following steps: loading Pd on an active carbon to obtain a Pd/carbon catalyst; mixing the Pd/carbon catalyst with a solvent to obtain a slurry; adding a sulfide to the slurry, stirring thoroughly at a predetermined temperature; filtering to obtain the sulfur-containing Pd/carbon catalyst. Through the preparation method Pd is initially loaded on the active carbon, and then reacts with the sulfide while loaded on the active carbon so as to be passivated partly. As a result, the use of the catalyst reduces side products produced during the preparation process of the PPD rubber antioxidant. It is unexpected to discover that the sulfide can hardly convert the metal Pd loaded on active carbon into palladium sulfide completely, and the sulfur is only selectively adsorbed and combined on the surfaces of metal Pd particles and various palladium sulphides are formed. Because of the selective adsorption and various palladium sulfides, the activity of Pd loaded on active carbon is partly passivated, thus it has solved the problem of low target product selectively caused by serious C-N bond hydrogenolysis side reactions in the raw materials and products.

The step of "loading Pd on an active carbon" mentioned in the preparation process of the present invention belongs to the prior art, and a conventional loading method may be applied. For example, in a specific embodiment provided by the present invention, the Pd/carbon catalyst may be prepared by the following method: weighing an active carbon used for preparing the catalyst, preparing the active carbon to a slurry at a temperature of 60°C to 90 °C, slowly adding a H₂PdCl₄ solution in a dropwise manner according to the Pd loading amount, stirring thoroughly and mixing uniformly, after an immersion of 0.5 to 5h, adjusting the pH value of the solution to 7.1 to 9, reducing the temperature to room temperature, filtering, washing a filter cake to be neutral with deionized water, then preparing the filter cake to a slurry at 20 °C to 90 °C, adding dropwise a liquid phase reduction agent to enable a reduction reaction, then filtering to obtain the Pd/carbon catalyst. The reduction agent in the reduction step may be formaldehyde, methanol, formic acid, or an alkali metal salt of the formic acid or hydrazine hydrate. Preferably, in the reduction reaction process, the molar ratio of the reduction agent to Pd is 2 to 200:1, preferably 5 to 50:1. Preferably, the temperature for reduction reaction is 20 °C to 100°C, and more preferably, about 30 °C to 80 °C. When the reduction is conducted in the preferred conditions, the reduction effect is more obvious, and most of the Pd ions loaded on the active carbon can be reduced to Pd.

It should be noted that the loading amount of Pd in the Pd/carbon catalyst prepared is preferably controlled within 0.5 to 10wt%, and more preferably about 1 to 5wt%, because the loading amount of the Pd affects subsequent reactions in that, if the loading amount of Pd is too low, longer time is required for preparing the PPD rubber antioxidant, which is not suitable for industrial production; if the loading amount of the Pd is too high, the stereoselectivity of a possible product is affected. Nevertheless, a sulfur-containing Pd/carbon catalyst beyond this range still functions well in improving the yield and selectivity of the PPD rubber antioxidant and preventing the raw materials from being reduced to the corresponding alcohols.

The loading amount of Pd may be precisely determined by the prior art. For example, a standard method for determining the loading amount of Pd on a Pd/carbon catalyst is provided in the National Standard of the P.R. China, GB/T 23518-2009.

Preferably, the granularity of active carbon adopted in the present invention is 50 to 1000 meshes, preferably 80 to 500 meshes; and the special surface area is 600 to 1800m²/g, and preferably 1000 to 1500m²/g. In the ranges for the granularity and the special surface area, the loading of Pd on active carbon is easier, and the distribution of the loaded Pd is relatively uniform.

After the Pd/carbon catalyst is obatained, the Pd/carbon catalyst is mixed with a solvent to form a slurry. In the step, the goal is to make the Pd/carbon catalyst into the slurry. Preferably, the solvent adopted here has certain polarity so as to optimize the dispersion of the Pd/carbon catalyst. For example, the solvent adopted in a specific embodiment provided by the present invention may be an alcohol, a ketone, or water, e.g. methanol, ethanol, acetone, MIBK, isopropanol, tert-butanol, isoamyl ketone, octanone, or water. It can be seen from the applicable solvents that the solvent adopted by the preparation method varies, and there is no stringent requirement for the specific solvent. In addition, the adopted solvent is cheap and very suitable for industrial production.

In addition, during the step of mixing the Pd/carbon catalyst with the solvent to form the slurry, preferably, the volume ratio of the Pd/carbon catalyst to the solvent is controlled within 1:5 to 1000, and preferably 10 to 400:1. If the volume of the solvent is too small, it is difficult to form the slurry; if the volume of the solvent is too big, energy is wasted and there is a problem of post-treatment of the solvent.

A essential step of the present invention is a sulfurizing step and the Pd/carbon catalyst can be prepared through the step. The sulfurizing step includes: after preparing the slurry, adding the sulfide to the slurry, and stirring thoroughly at a predetermined temperature. After this step, the sulfide and Pd loaded on the Pd/carbon catalyst react thoroughly so that Pd is partly passivated, and thus, the risk of Pd acting on the C-N bonds in the raw materials or products to cause cracking of the bonds and formation of numerous side products is reduced. Preferably, the molar ratio of the added sulfide to Pd loaded on the Pd/carbon catalyst is 0.1 to 10:1, and more preferably, 0.1 to 1:1. Within the preferred range, active carbon exhibits good catalytic performance after the sulfurization, and the hydrogenolysis side reactions can be well controlled. Of course, it can be seen from a specific embodiment provided by the present invention that, active carbon beyond the preferred range also exhibits controllability on the hydrogenolysis side reactions.

The sulfide mentioned the present invention refers such a sulfide: a sulfurizing agent that reacts with metal Pd to have adsorption and coordination effects so as to reduce the catalytic activity of metal Pd. Sulfur atoms in the sulfurizing agent must have lone pair electrons so that the sulfurizing agent may have the coordination effect with the metal Pd. Therefore, in principle, the sulfurizing agent adopted in the present invention can be a sulfide as long as the sulfide has sulfur atoms that have lone pair electrons. Preferably, the sulfide is selected from one or more in a group consisting of thiol, thioether, alkyl disulfide, thiofuran, hydrogen sulfide, ammonium sulfide, ammonium hydrosulfide, a sulfide of an alkaline metal, or a hydrosulfide of an alkaline metal. More preferably, the sulfide is selected from one or more of a group consisting of methyl mercaptan, ethyl thiol, methyl thioether, ethyl thioether, diphenyl thioether, dimethyl disulfide, thiofuran, hydrogen sulfide, ammonium sulfide, ammonium hydrosulfide, sodium sulfide, and potassium hydrosulfide.

In a specific embodiment provided by the present invention, when the sulfide is diphenyl thioether or dimethyl disulfide, the performance is superior to that of other substances.

The predetermined temperature in the reaction process above may be 20 °C to 100°C, preferably 30 °C to 70 °C. Further, the time for the sulfurizing treatment is longer than 10 minutes, preferably 2 to 5h. Therefore, it can be seen that the reaction provided by the present invention may be accomplished within a wide range of temperature, and the time for the sulfurizing treatment is very short, and the reaction may be generally accomplished in merely longer than 10 minutes. Therefore, energy consumption may be greatly reduced and product cost may be further reduced by industrial application of the method.

In a specific embodiment a specific process of the sulfurizing treatment step includes: after the reduced Pd/carbon catalyst is filtered, a filter cake is washed to be neutral with deionized water and vacuum dried at 60 °C to 110 °C. Then, it is ready for the sulfurizing treatment. During the step of the sulfurizing treatment, the reduced Pd/carbon catalyst and the solvent are first prepared to a slurry at 20 °C to 100 °C, and then, the sulfide is added at a molar ratio of Pd to the sulfide of 1:0.1 to 10, and the stirring time is longer than 10 minutes. Then, the step of sulfurization is accomplished.

After the sulfurization step, a filtrate may be filtered directly and a filter cake is dried to prepare the sulfur-containing Pd/carbon catalyst. The filter cake may be also vacuum dried at 30°C to 110 °C to completely remove the residual solvent in the catalyst. If the solvent in the sulfurization process may be introduced in the process for making the PPD antioxidant, it is unnecessary to vacuum dry the catalyst.

The sulfur-containing Pd/carbon catalyst provided is applied in the preparation method of the PPD rubber antioxidant. It is known to all that the PPD rubber antioxidant can be synthesized by many methods, including reduction alkylation, phenol-amine condensation, hydroxylamine reduction alkylation and quinone-imine condensation commonly used at present, while the catalyst provided by the present invention is applicable to the reaction for preparing the PPD antioxidant through reduction alkylation, e.g. the catalyst provided by the present invention may be applied to preparing products including 6PPD, N-isopropyl-N'-phenyl para-phenylene diamine (IPPD), N-(1,4-dimethyl pentyl)-N'-phenyl para-phenylene diamine (7PPD), N,N'-di(1,4-imethylpentyl) para-phenylene diamine (77PD), N-sec octyl-N'-phenyl para-phenylene diamine (OPPD), N,N'-di-sec-butyl para-phenylene diamine (44PD), N-isoamyl-N'-phenyl para-phenylene diamine (5PPD), N,N'-di(1,3-dimethylbutyl) para-phenylene diamine (66PD), and 2,4,6-tri-(N-1,4-dimethylpentyl-para-phenylene diamine)-1,3,5-triazine (TMPPD).

The reduction alkylation has one-step and two-step procedures. When the catalyst is applied in a preparation process with the one-step procedure, the preparation method of the PPD antioxidant includes the following steps: using 4-ADPA and an aliphatic ketone as raw materials, directly adding the sulfur-containing Pd/carbon catalyst to the reaction system while stirring, and introducing a predetermined amount of hydrogen to synthesize the PPD rubber antioxidant through liquid phase hydrogenation.

When the catalyst is applied in a preparation process with the two-step procedure, the preparation method of the PPD antioxidant includes the following steps: using 4-ADPA and an aliphatic ketone as raw materials and a proton acid or active carbon as a catalyst, performing dehydration condensation at 120°C to 150°C while stirring to synthesize an intermediate product; adding the Pd/carbon catalyst to the reaction system formed by the intermediate product and a solvent, then introducing a predetermined amount of hydrogen to synthesize the PPD rubber antioxidant through liquid phase hydrogenation.

In a specific embodiment provided by the present invention, the catalyst is applied to preparation of an antioxidant 4020, and a one-step procedure includes the following specific process: while stirring, using RT base (4-ADPA) and MIBK in excess as raw materials, and sulfurized Pd/active carbon as a catalyst to synthesize the rubber antioxidant 4020 through liquid phase hydrogenation at 90 °C to 240 °C and a hydrogen pressure of 1 to 5 MPa. A two-step procedure includes the following specific process: while stirring, using RT base and MIBK in excess as raw materials, a proton acid or active carbon as a catalyst to synthesize an imine through dehydration condensation at 120°C to 150°C; subjecting the generated imine to liquid phase hydrogenation at 90 °C to 220 °C and a hydrogen pressure of 1 to 5 MPa by using MIBK as a solvent and sulfurized Pd/active carbon as a catalyst to synthesize the rubber antioxidant 4020.

Preferably, the molar ratio of the RT base to MIBK is 1:2 to 10, preferably 1:2 to 6. At such molar ratios, the reaction is more thorough and applicable to mass industrial production.

Preferably, the use amount of the catalyst is 0.01 to 1wt% of the mass of the RT base on the mass of Pd, preferably 0.02 to 0.2wt%. At such preferred ratios, the reaction can be performed thoroughly, and controlling the content of the catalyst in the preferred range can further reduce the production and preparation cost.

Preferably, the temperature of the one-step procedure is preferably 100°C to 200 °C, and the hydrogen pressure is preferably 1.5 to 3MPa.

Preferably, the dehydration reaction temperature in the two-step procedure is preferably 120°C to 140°C, the hydrogenation reaction temperature is preferably 100°C to 200 °C, and the hydrogen pressure is preferably 1.5 to 3MPa.

Specific preparation methods of sulfur-containing Pd/carbon catalysts and beneficial effect brought about by applying the prepared catalysts in PPD antioxidants will be further described through embodiments hereinafter. However, the present invention is not limited to the following embodiments.

### Embodiment 1

Weigh 10g active carbon with a granularity of 100 meshes, and a special surface area of 1200 m²/g, add the active carbon to 100ml deionized water to prepare a slurry at a temperature of 80 °C, slowly add 10ml H₂PdCl₄ solution (Pd content is 0.05g/ml) in a dropwise manner, stir for 2h, adjust the pH value of the solution to 8 with 10wt% NaOH solution, reduce the temperature to room temperature, filter, and wash a filter cake to be neutral with deionized water, then prepare the filter cake to a slurry at 40 °C, dropwise add 2ml hydrazine hydrate solution having a concentration of 85%, stir for 2h, filter, wash a filter cake to be neutral with deionized water, dry the filter cake at 100°C in a vacuum condition, perform sulfurization for the filter cake, prepare the filter cake to a slurry at 40 °C with 100ml methanol, dropwise add 0.1ml methyl mercaptan, stir for 2h, filter, dry a filter cake at 100 °C in a vacuum condition to obtain a sulfurized Pd-active carbon catalyst.

### Embodiment 2

Weigh 10g active carbon with a granularity of 200 meshes, and a special surface area of 1400 m²/g, add the active carbon to 100ml deionized water to prepare a slurry at a temperature of 60°C, slowly add 6ml H₂PdCl₄ solution (Pd content is 0.05g/ml) in a dropwise manner, stir for 3h, adjust the pH value of the solution to 8.5 with 10wt% NaOH solution, reduce the temperature to room temperature, filter, and wash a filter cake to be neutral with deionized water, then prepare the filter cake to a slurry at 60°C, dropwise add 10ml methanol, stir for 4h, filter, wash a filter cake to be neutral with deionized water, dry the filter cake at 90°C in a vacuum condition, perform sulfurization for the filter cake, prepare the filter cake to a slurry at 60°C with 150ml ethanol, dropwise add 0.1ml diphenyl thioether, stir for 4h, filter, dry a filter cake at 80°C in a vacuum condition to obtain a sulfurized Pd-active carbon catalyst.

### Embodiment 3

Weigh 10g active carbon with a granularity of 150 meshes, and a special surface area of 1400 m²/g, add the active carbon to 100ml deionized water to prepare a slurry at a temperature of 60°C, slowly add 20ml H₂PdCl₄ solution (Pd content is 0.05g/ml) in a dropwise manner, stir for 4h, adjust the pH value of the solution to 9 with 10wt% NaOH solution, reduce the temperature to room temperature, filter, and wash a filter cake to be neutral with deionized water, then prepare the filter cake to a slurry at 60°C, dropwise add 35ml formaldehyde having a concentration of 40wt%, stir for 4h, filter, wash a filter cake to be neutral with deionized water, dry the filter cake at 90°C in a vacuum condition, perform sulfurization for the filter cake, prepare the filter cake to a slurry at 60°C with 200ml acetone, dropwise add 0.8ml thiofuran, stir for 4h, filter, dry a filter cake at 90°C in a vacuum condition to obtain a sulfurized Pd-active carbon catalyst.

### Embodiment 4

Weigh 10g active carbon with a granularity of 400 meshes, and a special surface area of 1600 m²/g, add the active carbon to 100ml deionized water to prepare a slurry at a temperature of 60°C, slowly add 4ml H₂PdCl₄ solution (Pd content is 0.05g/ml) in a dropwise manner, stir for 2h, adjust the pH value of the solution to 7.5 with 10wt% NaOH solution, reduce the temperature to room temperature, filter, and wash a filter cake to be neutral with deionized water, then prepare the filter cake to a slurry at 30°C, dropwise add 15ml formic acid having a concentration of 40wt%, stir for 4h, filter, wash a filter cake to be neutral with deionized water, dry the filter cake at 90°C in a vacuum condition, perform sulfurization for the filter cake, prepare the filter cake to a slurry at 60°C with 50ml MIBK, dropwise add 0.1ml dimethyl disulfide, stir for 4h, filter, dry a filter cake at 110°C in a vacuum condition to obtain a sulfurized Pd-active carbon catalyst.

### Embodiment 5

Weigh 10g active carbon with a granularity of 300 meshes, and a special surface area of 1600 m²/g, add the active carbon to 100ml deionized water to prepare a slurry at a temperature of 60°C, slowly add 10ml H₂PdCl₄ solution (Pd content is 0.05g/ml) in a dropwise manner, stir for 2h, adjust the pH value of the solution to 8.5 with 10wt% NaOH solution, reduce the temperature to room temperature, filter, and wash a filter cake to be neutral with deionized water, then prepare the filter cake to a slurry at 30°C, dropwise add 3ml hydrazine hydrate having a concentration of 85wt%, stir for 4h, filter, wash a filter cake to be neutral with deionized water, dry the filter cake at 90°C in a vacuum condition, perform sulfurization for the filter cake, prepare the filter cake to a slurry at 60°C with 200ml water, slowly introduce 0.5ml hydrogen sulfide gas in the form of bubbles, stir for 4h, filter, and dry a filter cake at 100°C in a vacuum condition to obtain a sulfurized Pd-active carbon catalyst.

### Embodiment 6

Weigh 10g active carbon with a granularity of 250 meshes, and a special surface area of 1600 m²/g, add the active carbon to 100ml deionized water to prepare a slurry at a temperature of 60°C, slowly add 10ml H₂PdCl₄ solution (Pd content is 0.05g/ml) in a dropwise manner, stir for 2h, adjust the pH value of the solution to 8.5 with 10wt% NaOH solution, reduce the temperature to room temperature, filter, and wash a filter cake to be neutral with deionized water, then prepare the filter cake to a slurry at 30°C, dropwise add 3ml hydrazine hydrate having a concentration of 85wt%, stir for 4h, filter, wash a filter cake to be neutral with deionized water, dry the filter cake at 90°C in a vacuum condition, perform sulfurization for the filter cake, prepare the filter cake to a slurry at 60°C with 200ml water, dropwise add 7ml sodium sulfide solution (1.1g/cm³,) having a concentration of at 10wt%, stir for 4h, filter, and dry a filter cake at 100°C in a vacuum condition to obtain a sulfurized Pd-active carbon catalyst.

### Embodiments 7 to 13

The preparation methods in the embodiments 7-13 are the same as the preparation method in the embodiment 1, except that the applied active carbon, the loading amount of Pd, the sulfide, the molar ratio of the sulfide to Pd, and the solvent adopted in the slurry were different. The conditions are recorded in details in Table 1 and the preparation processes will not be repeated here.

Embodiments 14 to 26 are embodiments of application of the Pd catalysts prepared by the preparation methods above in catalytic synthesis:

### Embodiment 14

Add 0.74g Pd catalyst prepared in the embodiment 1, 73.6g RT base, and 200ml MIBK to a 500ml stainless steel high-pressure reaction kettle, close the reaction kettle, replace the air inside the reaction kettle with nitrogen for three times and then with hydrogen for three times, increase the temperature to 140°C, wherein the hydrogen pressure was 2MPa, start stirring at 900r/min, react for 4h, end the reaction, take out the reaction solution after the temperature was reduced to room temperature, filter to remove the catalyst, analyze a filtrate with gas chromatography, wherein the results showed that the conversion rate of RT base was 99.5%, the selectivity of 4020 was 99.5%, and the selectivity of the MIBK was 99.8%.

### Embodiment 15

Add 0.55g Pd catalyst prepared in the embodiment 2, 55.2g RT base, and 187ml MIBK to a 500ml stainless steel high-pressure reaction kettle, close the reaction kettle, replace the air inside the reaction kettle with nitrogen for three times and then with hydrogen for three times, increase the temperature to 160°C, wherein the hydrogen pressure was 3MPa, start stirring at 900r/min, react for 5h, end the reaction, take out the reaction solution after the temperature was reduced to room temperature, filter to remove the catalyst, analyze a filtrate with gas chromatography, wherein the results showed that the conversion rate of RT base was 99.8%, the selectivity of 4020 was 98.7%, and the selectivity of the MIBK was 99.7%.

### Embodiment 16

Add 0.74g Pd catalyst prepared in the embodiment 3, 73.6g RT base, and 150ml MIBK to a 500ml stainless steel high-pressure reaction kettle, close the reaction kettle, replace the air inside the reaction kettle with nitrogen for three times and then with hydrogen for three times, increase the temperature to 100°C, wherein the hydrogen pressure was 3MPa, start stirring at 900r/min, react for 3h, end the reaction, take out the reaction solution after the temperature was reduced to room temperature, filter to remove the catalyst, analyze a filtrate with gas chromatography, wherein the results showed that the conversion rate of RT base was 99.3%, the selectivity of 4020 was 99.5%, and the selectivity of the MIBK was 99.9%.

### Embodiment 17

Add 0.55g Pd catalyst prepared in the embodiment 4, 55.2g RT base, and 225ml MIBK to a 500ml stainless steel high-pressure reaction kettle, close the reaction kettle, replace the air inside the reaction kettle with nitrogen for three times and then with hydrogen for three times, increase the temperature to 160°C, wherein the hydrogen pressure was 1MPa, start stirring at 900r/min, react for 6h, end the reaction, take out the reaction solution after the temperature was reduced to room temperature, filter to remove the catalyst, analyze a filtrate with gas chromatography, wherein the results showed that the conversion rate of RT base was 99.4%, the selectivity of 4020 was 99.4%, and the selectivity of the MIBK was 99.6%.

### Embodiment 18

Add 1.5g Pd catalyst prepared in the embodiment 5, 55.2g RT base, and 225ml MIBK to a 500ml stainless steel high-pressure reaction kettle, close the reaction kettle, replace the air inside the reaction kettle with nitrogen for three times and then with hydrogen for three times, increase the temperature to 220°C, wherein the hydrogen pressure was 2.5MPa, start stirring at 900r/min, react for 4h, end the reaction, take out the reaction solution after the temperature was reduced to room temperature, filter to remove the catalyst, analyze a filtrate with gas chromatography, wherein the results showed that the conversion rate of RT base was 98.9%, the selectivity of 4020 was 99.5%, and the selectivity of the MIBK was 99.2%.

### Embodiment 19

Add 55.2g RT base, 225ml MIBK and 2g active carbon to a 500 ml round bottomed flask with a water segregator, increase the reaction temperature to 130°C and perform dehydration condensation for 4h while stirring, add an imine obtained by the condensation reaction, 150ml MIBK and 0.55g Pd catalyst prepared in the embodiment 6, to a 500ml stainless steel high-pressure reaction kettle, close the reaction kettle, replace the air inside the reaction kettle with nitrogen for three times and then with hydrogen for three times, increase the temperature to 200°C, wherein the hydrogen pressure was 2MPa, start stirring at 900r/min, react for 4h, end the reaction, take out the reaction solution after the temperature was reduced to room temperature, filter to remove the catalyst, analyze a filtrate with gas chromatography, wherein the results showed that the conversion rate of RT base was 99.0%, the selectivity of 4020 was 99.6%, and the selectivity of the MIBK was 99.1 %.

### Embodiments 20 to 26

Specific operation processes in the embodiments 20 to 26 are the same as those of the embodiment 14 to 19, except that the catalysts adopted are different and the reaction processes may be one-step procedures or two-step procedures. Specific conditions are as shown in Table 2, and the operation processes will not be repeated here.

### Embodiment 27 (Preparation of IPPD)

Add 0.74g Pd catalyst prepared in the embodiment 1, 92g RT base and 51ml acetone to a 500 ml stainless steel high-pressure reaction kettle, close the reaction kettle, replace the air inside the reaction kettle with nitrogen for three times and then with hydrogen for three times, increase the temperature to 150°C, wherein the hydrogen pressure was 3.0MPa, start stirring at 900r/min, react for 4h, end the reaction, take out the reaction solution after the temperature was reduced to room temperature, filter to remove the catalyst, analyze a filtrate with gas chromatography, wherein the results showed that the conversion rate of RT base was 99.5%, the selectivity of IPPD was 99.6%, and the selectivity of the acetone was 99.8%.

The comparative example 1 and the comparative example 2 are examples of application of unsulfurized Pd/carbon catalysts in catalyzing and synthesizing 4020.

### Comparative example 1

Add 1.5g unsulfurized Pd/carbon catalyst having a loading amount of 5%, 55.2g RT base and 225ml MIBK to a 500ml stainless steel high-pressure reaction kettle, close the reaction kettle, replace the air inside the reaction kettle with nitrogen for three times and then with hydrogen for three times, increase the temperature to 160°C, wherein the hydrogen pressure was 2.5MPa, start stirring at 900r/min, react for 4h, end the reaction, take out the reaction solution after the temperature was reduced to room temperature, filter to remove the catalyst, analyze a filtrate with gas chromatography, wherein the results showed that the conversion rate of RT base was 99.4%, the selectivity of 4020 was 81.6%, and the selectivity of the MIBK was 99.1%.

### Comparative Example 2

Add 55.2g RT base, 225ml MIBK and 2g active carbon to a 500 ml round bottomed flask with a water segregator, increase the reaction temperature to 130°C and perform dehydration condensation for 4h while stirring, add an imine obtained by the condensation reaction, 150ml MIBK and 0.55g unsulfurized Pd/carbon catalyst with a loading amount of 3% to a 500ml stainless steel high-pressure reaction kettle, close the reaction kettle, replace the air inside the reaction kettle with nitrogen for three times and then with hydrogen for three times, increase the temperature to 180°C, wherein the hydrogen pressure was 2MPa, start stirring at 900r/min, react for 4h, end the reaction, take out the reaction solution after the temperature was reduced to room temperature, filter to remove the catalyst, analyze a filtrate with gas chromatography, wherein the results showed that the conversion rate of RT base was 99.8%, the selectivity of 4020 was 75.2%, and the selectivity of the MIBK was 99.2%.

**Table 1 Sulfurized Pd-active carbon catalysts provided by Embodiments 1 to 13**

| | Active carbon | Pd loading amount | Molar ratio of sulfide to Pd | Sulfide | Slurry solvent |
|---|---|---|---|---|---|
| Embodiment 1 | 100 meshes 1200m²/g | 5wt% | 0.38:1 | Methyl mercaptan | Methanol |
| Embodiment 2 | 200 meshes 1400m²/g | 3wt% | 0.21:1 | Diphenyl thioether | Ethanol |
| Embodiment 3 | 150 meshes 1400m²/g | 10wt% | 1.05:1 | Thiofuran | Acetone |
| Embodiment 4 | 400 meshes 1600m²/g | 2wt% | 0.58:1 | Dimethyl disulfide | MIBK |
| Embodiment 5 | 300 meshes 1600m²/g | 5wt% | - | Hydrogen sulfide gas | Water |
| Embodiment 6 | 250 meshes 1600m²/g | 5wt% | 2.1:1 | Sodium sulfide | Water |
| Embodiment 7 | 50 meshes 600m²/g | 0.5wt% | 0.1:1 | Ethyl thiol | Ethyl acetate |
| Embodiment 8 | 1000 meshes 1800m²/g | 0.3wt% | 5:1 | Ethyl thioether | Ethanol |
| Embodiment 9 | 1200 meshes 2000m²/g | 15wt% | 10:1 | Ammonium sulfide | Water |
| Embodiment 10 | 200 meshes 1400m²/g | 4wt% | 11:1 | Ammonium hydrosulfide | Water |
| Embodiment 11 | 150 meshes 1400m²/g | 8wt% | 0.05:1 | Potassium hydrosulfide | Water |
| Embodiment 12 | 250 meshes 1600m²/g | 5wt% | 4:1 | Methyl mercaptan and diphenyl thioether in a ratio of 2:1 | Methanol |
| Embodiment 13 | 50 meshes 600m²/g | 0.5wt% | 8:1 | Ammonium sulphide and potassium hydrosulfide in a ratio of 1:1 | Water |

**Table 2 Influence of catalysts provided by the present invention and the comparative examples on reactions**

| | Catalyst | Reaction procedure | Amount of catalyst | conversion rate of RT base | selectivity of 4020 | selectivity of MIBK |
|---|---|---|---|---|---|---|
| Embodiment 14 | Embodiment 1 | One-step procedure | 0.05wt% | 99.5% | 99.5% | 99.8% |
| Embodiment 15 | Embodiment 2 | One-step procedure | 0.03wt% | 99.8% | 99.7% | 99.7% |
| Embodiment 16 | Embodiment 3 | One-step procedure | 0.1 wt% | 99.3% | 99.5% | 99.9% |
| Embodiment 17 | Embodiment 4 | Two-step procedure | 0.02wt% | 99.4% | 99.8% | 99.6% |
| Embodiment 18 | Embodiment 5 | Two-step procedure | 0.14wt% | 98.9% | 99.5% | 99.2% |
| Embodiment 19 | Embodiment 6 | Two-step procedure | 0.05wt% | 99.0% | 99.6% | 99.1% |
| Embodiment 20 | Embodiment 7 | One-step procedure | 0.2wt% | 99.1% | 99.4% | 99.7% |
| Embodiment 21 | Embodiment 8 | One-step procedure | 0.5wt% | 99.4% | 99.6% | 99.5% |
| Embodiment 22 | Embodiment 9 | One-step procedure | 0.6wt% | 98.9% | 99.3% | 99.7% |
| Embodiment 23 | Embodiment 10 | One-step procedure | 1.00wt% | 99.1% | 99.3% | 99.7% |
| Embodiment 24 | Embodiment 11 | Two-step procedure | 0.005wt% | 99.3% | 99.2% | 99.2% |
| Embodiment 25 | Embodiment 12 | Two-step procedure | 2.00wt% | 99.3% | 99.4% | 99.8% |
| Embodiment 26 | Embodiment 13 | Two-step procedure | 0.8wt% | 99.5% | 99.2% | 99.8% |
| Embodiment 27 | Embodiment 1 | One-step procedure | 0.05wt% | 99.5% | 99.6% | 99.8% |
| Comparative example 1 | Unsulfurized Pd/carbon catalyst | One-step procedure | 0.13wt% | 99.4% | 81.6% | 99.1% |
| Comparative example 2 | Unsulfurized Pd/carbon catalyst | Two-step procedure | 0.03wt% | 99.8% | 75.2% | 99.2% |

Compared with the prior art, the present invention has the following advantages:
1) high activity and high selectivity, the present invention applies a sulfurized Pd/carbon catalyst to synthesis of an PPD rubber antioxidant, e.g. a reaction of rubber antioxidant 4020, exhibiting extremely high activity and selectivity, with a RT base conversion rate as high as 99.8%, a 4020 selectivity as high as 99.5%, and an MIBK selectivity higher than 99.5%;
2) low cost, the main component of a catalyst used in the present invention is Pd, and the loading amount of Pd is similar to that of platinum in a platinum/carbon catalyst; therefore, the sulphur-containing Pd/carbon catalyst, with higher economic benefit, is cheaper than the traditional platinum/carbon catalyst.

## Claims

1. Preparation method for a p-phenylenediamine antioxidant, using 4-aminodiphenylamine and an aliphatic ketone as raw materials to prepare the PPD antioxidant, **characterized in that** a sulfur-containing Pd/carbon catalyst is added to the reaction system as a catalyst, and the sulfur-containing Pd/carbon catalyst is prepared by the preparation method comprising the following steps:
loading Pd onto activated carbon, and obtaining a Pd/carbon catalyst;
preparing a slurry by mixing the Pd/carbon catalyst with a solvent;
adding a sulfide into the slurry, and stirring at a predetermined temperature;
filtering, and obtaining the sulfur-containing Pd/carbon catalyst;
wherein the predetermined temperature is 20 °C to 100 °C.

2. Preparation method according to claim 1, **characterized in that** the catalyst is added to the reaction system in an amount such that the weight ratio of Pd loaded on the sulfur-containing Pd/carbon catalyst to 4-ADPA is 0.01 to 1 %.

3. Preparation method according to claim 1, **characterized in that** the preparation method comprises the following steps:
using 4-ADPA and the aliphatic ketone as the raw materials, adding the sulfur-containing Pd/carbon catalyst into the reaction system while stirring, introducing hydrogen, and synthesizing the PPD antioxidant through liquid phase hydrogenation.

4. Preparation method according to claim 1, **characterized in that** the preparation method comprises the following steps:
using 4-ADPA and the aliphatic ketone as the raw materials and a protonic acid or activated carbon, performing dehydration condensation at 120°C to 150°C while stirring to synthesize an intermediate product;
adding the Pd/carbon catalyst into a reaction system formed by the intermediate product and a solvent, introducing hydrogen, and synthesizing the PPD antioxidant through liquid phase hydrogenation.

5. Preparation method according to claim 1, **characterized in that** in the step of adding the sulfide into the slurry, the molar ratio of the added sulfide to Pd loaded on the Pd/carbon catalyst is 0.1 to 10:1.

6. Preparation method according to claim 5, **characterized in that** the molar ratio of the added sulfide to Pd loaded on the Pd/carbon catalyst is 0.1 to 1:1.

7. Preparation method according to any one of claims 1, 5 and 6, **characterized in that** the sulfide is one or more selected from a group consisting of thiol, thioether, alkyl disulfide, thiofuran, hydrogen sulfide, ammonium sulfide, ammonium hydrosulfide, a sulfide of an alkaline metal, and a hydrosulfide of an alkaline metal.

8. Preparation method according to any one of claims 1, 5 and 6, **characterized in that** the sulfide is one or more selected from a group consisting of methyl mercaptan, ethyl thiol, methyl thioether, ethyl thioether, diphenyl thioether, dimethyl disulfide, thiofuran, hydrogen sulfide, ammonium sulfide, ammonium hydrosulfide, sodium sulfide, and potassium hydrosulfide.

9. Preparation method according to claim 8, **characterized in that** the sulfide is diphenyl thioether or dimethyl disulfide.

10. Preparation method according to claim 1, **characterized in that** the solvent is methanol, ethanol, acetone, methylisobutyl ketone or water.

11. Preparation method according to claim 1, **characterized in that** the granularity of activated carbon in the Pd/carbon catalyst is 50 to 1000 meshes; the specific surface area of the activated carbon is 600 to 1800 m²/g; and the loading amount of Pd is 0.5 to lOw.

## Patentansprüche

1. Herstellungsverfahren für ein p-Phenylendiamin-Antioxidans unter Verwendung von 4-Aminodiphenylamin und eines aliphatischen Ketons als Rohstoffe, um das PPD-Antioxidans herzustellen, **dadurch gekennzeichnet, dass** dem Reaktionssystem ein schwefelhaltiger Pd/Kohlenstoff-Katalysator als ein Katalysator zugegeben wird und der schwefelhaltige Pd/Kohlenstoff-Katalysator über das Herstellungsverfahren hergestellt wird, das die folgenden Schritte umfasst:
Laden von Pd auf Aktivkohle und Erhalten eines Pd/Kohlenstoff-Katalysators;
Herstellen einer Aufschlämmung durch Mischen des Pd/Kohlenstoff-Katalysators mit einem Lösungsmittel;
Zugeben eines Sulfids in die Aufschlämmung und Rühren bei einer vorbestimmten Temperatur;
Filtern und Erhalten des schwefelhaltigen Pd/Kohlenstoff-Katalysators;
Wobei die vorbestimmte Temperatur beträgt 20 °C bis 100 °C.

2. Herstellungsverfahren nach Anspruch 1, **dadurch gekennzeichnet, dass** der Katalysator dem Reaktionssystem in einer derartigen Menge zugegeben wird, dass das Gewichtsverhältnis von auf den schwefelhaltigen Pd/Kohlenstoff-Katalysator geladenem Pd zu 4-ADPA 0,01 bis 1 % beträgt.

3. Herstellungsverfahren nach Anspruch 1, **dadurch gekennzeichnet, dass** das Herstellungsverfahren die folgenden Schritte umfasst:
unter Verwendung von 4-ADPA und des aliphatischen Ketons als die Rohstoffe, Zugeben des schwefelhaltigen Pd/Kohlenstoff-Katalysators unter Rühren in das Reaktionssystem, Einleiten von Wasserstoff und Synthetisieren des PPD-Antioxidans durch Flüssigphasenhydrierung.

4. Herstellungsverfahren nach Anspruch 1, **dadurch gekennzeichnet, dass** das Herstellungsverfahren die folgenden Schritte umfasst:
unter Verwendung von 4-ADPA und des aliphatischen Ketons als die Rohstoffe und einer Protonensäure oder Aktivkohle, Durchführen einer Dehydrierungskondensation bei 120 °C bis 150 °C unter Rühren, um ein Zwischenprodukt zu synthetisieren;
Zugeben des Pd/Kohlenstoff-Katalysators in ein Reaktionssystem, das von dem Zwischenprodukt und einem Lösungsmittel gebildet wird, Einleiten von Wasserstoff und Synthetisieren des PPD-Antioxidans durch Flüssigphasenhydrierung.

5. Herstellungsverfahren nach Anspruch 1, **dadurch gekennzeichnet, dass** im Schritt des Zugebens des Sulfids in die Aufschlämmung das Molverhältnis des zugegebenen Sulfids zu auf den Pd/Kohlenstoff-Katalysator geladenem Pd 0,1 bis 10:1 beträgt.

6. Herstellungsverfahren nach Anspruch 5, **dadurch gekennzeichnet, dass** das Molverhältnis des zugegebenen Sulfids zu auf den Pd/Kohlenstoff-Katalysator geladenem Pd 0,1 bis 1:1 beträgt.

7. Herstellungsverfahren nach einem der Ansprüche 1, 5 und 6, **dadurch gekennzeichnet, dass** das Sulfid eines oder mehrere ist, ausgewählt aus einer Gruppe bestehend aus Thiol, Thioether, Alkyldisulfid, Thiofuran, Wasserstoffsulfid, Ammoniumsulfid, Ammoniumhydrosulfid, einem Sulfid eines Alkalimetalls und einem Hydrosulfid eines Alkalimetalls.

8. Herstellungsverfahren nach einem der Ansprüche 1, 5 und 6, **dadurch gekennzeichnet, dass** das Sulfid eines oder mehrere ist, ausgewählt aus einer Gruppe bestehend aus Methylmercaptan, Ethylthiol, Methylthioether, Ethylthioether, Diphenylthioether, Dimethyldisulfid, Thiofuran, Wasserstoffsulfid, Ammoniumsulfid, Ammoniumhydrosulfid, Natriumsulfid und Kaliumhydrosulfid.

9. Herstellungsverfahren nach Anspruch 8, **dadurch gekennzeichnet, dass** das Sulfid Diphenylthioether oder Dimethyldisulfid ist.

10. Herstellungsverfahren nach Anspruch 1, **dadurch gekennzeichnet, dass** das Lösungsmittel Methanol, Ethanol, Aceton, Methylisobutylketon oder Wasser ist.

11. Herstellungsverfahren nach Anspruch 1, **dadurch gekennzeichnet, dass** die Körnung der Aktivkohle im Pd/Kohlenstoff-Katalysator 50 bis 1000 Meshes beträgt; die spezifische Oberfläche der Aktivkohle 600 bis 1800 m²/g beträgt; und die Lademenge von Pd 0,5 bis 10 Gew.-% beträgt.

## Revendications

1. Procédé de préparation d'un antioxydant p-phénylènediamine, en utilisant de la 4-aminodiphénylamine et une cétone aliphatique en tant que matières premières pour préparer l'antioxydant PPD, **caractérisé en ce qu'**un catalyseur de Pd/carbone contenant du soufre est ajouté au système de réaction en tant que catalyseur, et le catalyseur de Pd/carbone contenant du soufre est préparé par le procédé de préparation comprenant les étapes suivantes :
chargement de Pd sur du carbone activé, et l'obtention d'un catalyseur de Pd/carbone ;
préparation d'une suspension en mélangeant le catalyseur de Pd/carbone avec un solvant ;
ajout d'un sulfure dans la suspension, et agitation à une température prédéterminée ;
filtrage, et obtention du catalyseur de Pd/carbone contenant du soufre ;
où la température prédéterminée est de 20 °C à 100 °C.

2. Procédé de préparation selon la revendication 1, **caractérisé en ce que** le catalyseur est ajouté au système de réaction en une quantité telle que le rapport en poids du Pd chargé sur le catalyseur de Pd/carbone contenant du soufre sur 4-ADPA soit de 0,01 à 1 %.

3. Procédé de préparation selon la revendication 1, **caractérisé en ce que** le procédé de préparation comprend les étapes suivantes :
utilisation de 4-ADPA et de la cétone aliphatique en tant que matières premières, ajout du catalyseur de Pd/carbone contenant du soufre dans le système de réaction en agitant, introduction d'hydrogène, et synthèse de l'antioxydant PPD par hydrogénation en phase liquide.

4. Procédé de préparation selon la revendication 1, **caractérisé en ce que** le procédé de préparation comprend les étapes suivantes :
utilisation de 4-ADPA et de la cétone aliphatique en tant que matières premières et d'un acide protonique ou de carbone activé, réalisation d'une déshydratation/condensation à une température de 120 °C à 150 °C en agitant pour synthétiser un produit intermédiaire ;
ajout du catalyseur de Pd/carbone dans un système de réaction formé par le produit intermédiaire et un solvant, introduction d'hydrogène, et synthèse de l'antioxydant PPD par hydrogénation en phase liquide.

5. Procédé de préparation selon la revendication 1, **caractérisé en ce que**, à l'étape d'ajout du sulfure dans la suspension, le rapport molaire du sulfure ajouté sur le Pd chargé sur le catalyseur de Pd/carbone est de 0,1 à 10:1.

6. Procédé de préparation selon la revendication 5, **caractérisé en ce que** le rapport molaire du sulfure ajouté sur le Pd chargé sur le catalyseur de Pd/carbone est de 0,1 à 1:1.

7. Procédé de préparation selon l'une quelconque des revendications 1, 5 et 6, **caractérisé en ce que** le sulfure est un ou plusieurs sélectionnés dans un groupe se composant du thiol, du thioéther, du disulfure d'alkyle, du thiofurane, du sulfure d'hydrogène, du sulfure d'ammonium, de l'hydrosulfure d'ammonium, d'un sulfure d'un métal alcalin, et d'un hydrosulfure d'un métal alcalin.

8. Procédé de préparation selon l'une quelconque des revendications 1, 5 et 6, **caractérisé en ce que** le sulfure est un ou plusieurs sélectionnés dans un groupe se composant du méthyle mercaptan, de l'éthyle thiol, du méthyle thioéther, de l'éthyle thioéther, du diphényle thioéther, du disulfure de diméthyle, du thiofurane, du sulfure d'hydrogène, du sulfure d'ammonium, de l'hydrosulfure d'ammonium, du sulfure de sodium, et de l'hydrosulfure de potassium.

9. Procédé de préparation selon la revendication 8, **caractérisé en ce que** le sulfure est du diphényle thioéther ou du disulfure de diméthyle.

10. Procédé de préparation selon la revendication 1, **caractérisé en ce que** le solvant est du méthanol, de l'éthanol, de l'acétone, de la cétone méthylisobutyle ou de l'eau.

11. Procédé de préparation selon la revendication 1, **caractérisé en ce que** la granularité du carbone activé dans le catalyseur de Pd/carbone est de 50 à 1000 Mesh ; l'aire de surface spécifique du carbone activé est de 600 à 1800 m²/g ; et la quantité de chargement de Pd est de 0,5 à 10 % en poids.
